# EUROPEAN PATENT APPLICATION

(11) **EP 1 927 312 A1**
(43) Date of publication of application: **04.06.2008**
(21) Application number: 06797082.2
(22) Date of filing: 30.08.2006
(51) Int. Cl.: A61B 1/00, G02B 23/24

(54) **ENDOSCOPE INSERTION PART**

(30) Priority: 22.09.2005 JP 2005276206; 10.03.2006 JP 2006065573
(71) Applicant: Olympus Corporation, Tokyo 151-0072 (JP)
(72) Inventor: KITAGAWA, Hideya, .. (JP); ADACHI, Katsutaka, .. (JP); SUZUKI, Takeo, .. (JP); NAKAURA, Yoshinori, .. (JP); SAKASHITA, Yoshihide, .. (JP)
(74) Representative: Winter, Brandl, Fürniss, Hübner Röss, Kaiser, Polte Partnerschaft Patent- und Rechtsanwaltskanzlei
(86) International application number: PCT/JP2006/317109
(87) International publication number: WO 2007/034664

(57) **Abstract**

An easy-to-manufacture endoscope is provided.

An endoscope insertion portion includes an inside tubular member (28a) shaped substantially a cylinder, and extended over at least a bending portion (20), an outside tubular member (28b) shaped substantially a cylinder, provided over an outside of the inside tubular member (28a), and extended over at least a bending portion (20), and an operation wire (36) for bending operation of the inside tubular member (28a) and outside tubular member (28b), wherein at least one tubular member (28a) of the inside tubular member (28a) and outside tubular member (28b) is formed as one body, and includes at least two cylindrical portions (31) and a joint portion (31) including elasticity and connecting the adjacent cylindrical portions to be bendable to each other, and a wire holding portion (34) is formed by the inside tubular member (28a) and outside tubular member (28b) and holds the operation wire (36) to be movable forward and backward between the inside tubular member (28a) and outside tubular member (28b).

## Description

### Technical Field

The present invention relates to an endoscope insertion portion with a bending portion for bending operation.

### Background Art

In a conventional endoscope, an elongated insertion portion is inserted into a body cavity for observation. Such an insertion portion includes a bending portion for bending operation. An example of such a bending portion is disclosed in Jpn. Pat. Appln. KOKAI Publication No. 5-3852. In the bending portion disclosed in the Jpn. Pat. Appln. KOKAI Publication No. 5-3852, substantially cylindrical bending parts are connected coaxially and rotatably with rivets. A wire holding portion is fixed to the inner periphery surface of each bending part by laser welding, for example. An operation wire is inserted through the wire holding portion of each bending part. The distal end of the operation wire is connected to the distal end portion of the bending portion, and the proximal end of the operation wire is connected to a bending operation mechanism within a control portion provided in the proximal end portion of the insertion portion. By moving the operation wire forward or backward by the bending operation mechanism, the bending parts are rotated to each other and the bending portion is bent.

### Disclosure of Invention

In the bending portion disclosed in Jpn. Pat. Appln. KOKAI Publication No. 5-3852, bending parts are connected rotatably with rivets, and wire holding portions are fixed to the inner periphery surface of the bending parts. Thus, in an endoscope with a particularly small diameter, it is necessary to precisely machine and assemble very small parts. Thus, a step of manufacturing a bending portion is very complex, and manufacturing of an endoscope is difficult.

The present invention has been made to solve the above problems. Accordingly, it is an object of the invention is to provide an easy-to-manufacture endoscope insertion portion.

In an aspect of the present invention, an endoscope insertion portion is characterized by including: an inside tubular member shaped substantially a cylinder, and extended over at least a bending portion; an outside tubular member shaped substantially a cylinder, provided over an outside of the inside tubular member, and extended over at least a bending portion; and an operation wire for bending operation of the inside tubular member and outside tubular member; wherein at least one tubular member of the inside tubular member and outside tubular member is formed as one body, and includes at least two cylindrical portions and a joint portion including elasticity and connecting the adjacent cylindrical portions to be bendable to each other, and a wire holding portion is formed by the inside tubular member and outside tubular member and holds the operation wire to be movable forward and backward between the inside tubular member and outside tubular member.

In a preferred aspect of the present invention, the endoscope insertion portion is characterized in that the other tubular member of the inside tubular member and outside tubular member is formed as one body, and includes at least two cylindrical portions and a joint portion including elasticity and connecting the adjacent cylindrical portions to be bendable to each other.

In a preferred aspect of the present invention, the endoscope insertion portion is characterized in that the wire holding portion is formed by a groove formed on the outer periphery surface of the inside tubular member and extended in a central axial direction of the inside tubular member and the inner periphery surface of the outside tubular member.

In a preferred aspect of the present invention, the endoscope insertion portion is characterized in that the wire holding portion is formed by the outer periphery surface of the inside tubular member and a groove formed on the inner periphery surface of the outside tubular member and extended in a central axial direction of the outside tubular member.

In a preferred aspect of the present invention, the endoscope insertion portion is characterized in that the wire holding portion is formed by an inside groove formed on the outer periphery surface of the inside tubular member and extended in a central axial direction of the inside tubular member and an outside groove formed on the inner periphery surface of the outside tubular member, extended in a central axial direction of the outside tubular member, and facing to the inside groove.

In a preferred aspect of the present invention, the endoscope insertion portion is characterized in that the joint portions of the other tubular member are successively and helically arranged in a central axial direction of the other tubular member.

In a preferred aspect of the present invention, the endoscope insertion portion is characterized in that the other tubular member of the inside tubular member and outside tubular member is formed by a helical tube formed as one body.

In a preferred aspect of the present invention, the endoscope insertion portion is characterized in that the helical tube is extended over the bending portion and the insertion tube portion.

In a preferred aspect of the present invention, the endoscope insertion portion is characterized in that the one tubular member of the inside tubular member and outside tubular member is extended over the bending portion, and the other tubular member of the inside tubular member and outside tubular member is extended over the bending portion and the insertion tube portion.

In a preferred aspect of the present invention, the endoscope insertion portion is characterized in that the inside tubular member is formed by cutting a base member including cylindrical portions provided coaxially to each other and joint portions connecting the adjacent cylindrical portions to be bendable to each other and formed continuously, into a predetermined length.

In a preferred aspect of the present invention, the endoscope insertion portion is characterized in that the outside tubular member is formed by cutting a base member including cylindrical portions provided coaxially to each other and joint portions connecting the adjacent cylindrical portions to be bendable to each other and formed continuously, into a predetermined length.

In a preferred aspect of the present invention, the endoscope insertion portion is characterized in that the inside tubular member is extended over the bending portion, the outside tubular member is extended over the bending portion and the insertion tube portion, the operation wire is inserted through the bending portion and the insertion tube portion, the endoscope insertion portion further comprises a sheath provided over an outside of the operation wire in the insertion tube portion, the inside tubular member includes at least one boss provided in a proximal end portion of the inside tubular member, connected to the wire holding portion, and connected to a distal end of the sheath, and the operation wire is inserted from the wire holding portion into the sheath through the boss.

In a preferred aspect of the present invention, the endoscope insertion portion is characterized in that the inside tubular member is extended over the bending portion and the insertion tube portion, and the outside tubular member is extended over the bending portion.

In a preferred aspect of the present invention, the endoscope insertion portion is characterized by further comprising an outer tube formed as one body and extended over the bending portion and the insertion tube portion.

In a preferred aspect of the present invention, the endoscope insertion portion characterized in that a length of the joint portion in the longitudinal axial direction of the tubular member in the bending portion is longer than a length of the joint portion in the longitudinal axial direction of the tubular member in the insertion tube portion.

In a preferred aspect of the present invention, the endoscope insertion portion is characterized in that a width of the joint portion in a peripheral direction of the tubular member in the bending portion is narrower than a width of the joint portion in the peripheral direction of the tubular member in the insertion tube portion.

In a preferred aspect of the present invention, the endoscope insertion portion is characterized in that a thickness of the outer tube in the bending portion is thinner than a thickness of the outer tube in the insertion tube portion.

In another aspect of the present invention, an endoscope is characterized by including the above endoscope insertion portion.

According to the invention, manufacturing of an endoscope insertion portion is easy.

### Brief Description of Drawings

FIG. 1 is a perspective view of an endoscope according to a first embodiment of the present invention;
FIG. 2 is a perspective view of an inside tubular member of the endoscope according to the first embodiment of the present invention;
FIG. 3 is a perspective view of an outside tubular member of the endoscope according to the first embodiment of the present invention;
FIG. 4A is a perspective view of a bending portion of the endoscope according to the first embodiment of the present invention;
FIG. 4B is a transverse cross-sectional view of the bending portion of the endoscope according to the first embodiment of the present invention cut off along IVB-IVB lines of FIG. 4A;
FIG. 4C is a longitudinal cross-sectional view of a structure of fixing the distal end of an operation wire of the endoscope according to the first embodiment of the present invention;
FIG. 4D is a transverse cross-sectional view of the structure of fixing the distal end of the operation wire of the endoscope according to the first embodiment of the present invention cut off along lines IVD-IVD of FIG. 4A;
FIG. 5A is a transverse cross-sectional view of a bending portion of an endoscope according to a second embodiment of the present invention;
FIG. 5B is a longitudinal cross-sectional view of a structure of fixing the distal end of an operation wire of the endoscope according to the second embodiment of the present invention;
FIG. 5C is a transverse cross-sectional view of the structure of fixing the distal end of the operation wire of the endoscope according to the second embodiment of the present invention;
FIG. 6A is a transverse cross-sectional view of a bending portion of an endoscope according to a third embodiment of the present invention;
FIG. 6B is a longitudinal cross-sectional view of a structure of fixing the distal end of an operation wire of the endoscope according to the third embodiment of the present invention;
FIG. 6C is a transverse cross-sectional view of the structure of fixing the distal end of the operation wire of the endoscope according to the third embodiment of the present invention;
FIG. 7 is a perspective view of a structure of fixing the distal end of an operation wire of an endoscope according to a fourth embodiment of the present invention;
FIG. 8 is a perspective view of an outside tubular member of an endoscope according to a fifth embodiment of the present invention;
FIG. 9 is a perspective view of a bending portion of an endoscope according to a sixth embodiment of the present invention;
FIG. 10 is an exploded perspective view of an endoscope insertion portion according to a seventh embodiment of the present invention;
FIG. 11A is an exploded perspective view of an endoscope insertion portion according to an eighth embodiment of the present invention;
FIG. 11B is a perspective view of the endoscope insertion portion of the eighth embodiment of the present invention;
FIG. 12 is a perspective view showing connection between a boss and a coil sheath of an endoscope according to a ninth embodiment of the present invention;
FIG. 13 is a perspective view showing connection between a boss and a coil sheath of an endoscope according to a first modification of the ninth embodiment of the present invention;
FIG. 14 is a perspective view showing connection between a boss and a coil sheath of an endoscope according to a second modification of the ninth embodiment of the present invention;
FIG. 15A is a top view of an endoscope insertion portion according to a tenth embodiment of the present invention;
FIG. 15B is a longitudinal cross-sectional view of the endoscope insertion portion according to the tenth embodiment of the present invention;
FIG. 15C is a transverse cross-sectional view of the endoscope insertion portion according to the tenth embodiment of the present invention;
FIG. 15D is a longitudinal cross-sectional view for explaining the effects of the endoscope according to the tenth embodiment of the present invention;
FIG. 16A is a longitudinal cross-sectional view of an insertion portion according to a first modification of the tenth embodiment of the present invention;
FIG. 16B is a transverse cross-sectional view of the insertion portion according to the first modification of the tenth embodiment of the present invention;
FIG. 17 is a top view of an endoscope insertion portion according to an eleventh embodiment of the present invention; and
FIG. 18 is a top view of an endoscope insertion portion according to a first modification of the eleventh embodiment of the present invention;

### Best Mode for Carrying Out the Invention

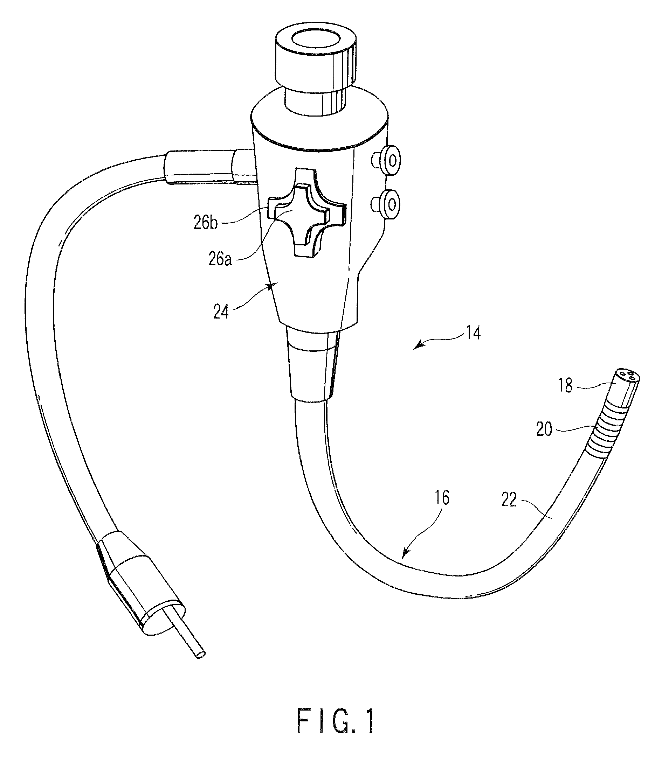

A first embodiment of the present invention will now be explained with reference to the accompanying drawings FIGS. 1 to 4D. As shown in FIG. 1, an endoscope 14 according to this embodiment includes an elongated insertion portion 16 to be inserted into a body cavity. The insertion portion 16 is formed by connecting a distal end forming portion 18, a bending portion 20 for bending operation and a long flexible insertion tube portion 22 successively from the distal end side. A control portion 24 to be held and operated by an operator is connected to the proximal end portion of the insertion portion 16. In the control portion 24, an up/down bending operation knob 26a and a left/right bending operation knob 26b are provided for bending operation of the bending portion 20.

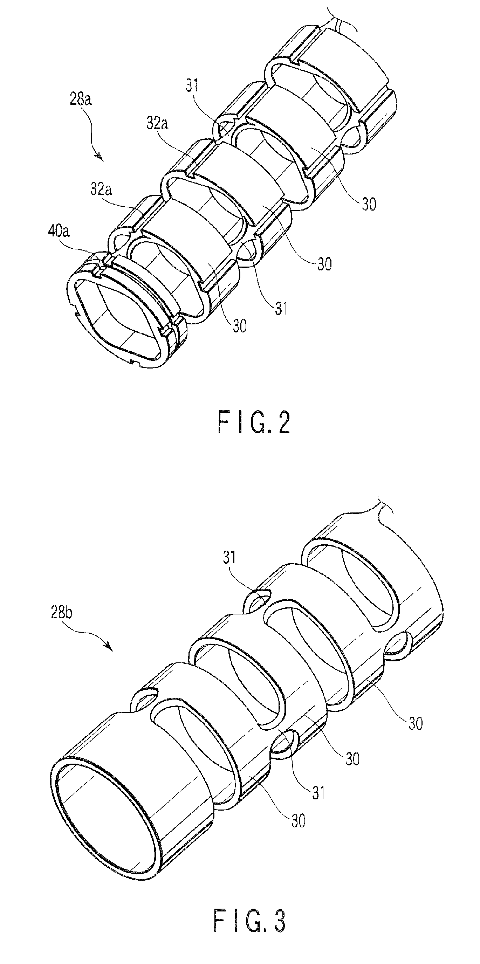

As shown in FIGS. 1 and 2, the bending portion 20 of this embodiment includes a substantially cylindrical inside tubular member 28a. The inside tubular member 28a is formed as one body by extrusion or injection molding of elastic resin material, or by pressing metal material.

In the inside tubular member 28a, a number of cylindrical portions 30 is arranged side by side coaxially to each other. Adjacent cylindrical portions are connected by a pair of elastic joint portions 31 symmetrical to each other with respect to the central axis of the inside tubular member 28a and rotatable to each other by bending deformation of the joint portion 31. Each pair of joint portions 31 is formed to be successively displaced 90° in the peripheral direction of the central axis of the inside tubular member 28a along the central axial direction. Therefore, the cylindrical portions 30 forward and backward a cylindrical portion 30 are rotatable in the direction perpendicular to each other. By combining such rotations, the inside tubular member 28a is bendable in any direction.

In the outer periphery surface of the inside tubular member 28a, an inside groove 32a is provided at up, down, left, and right positions in regard to an observation view field, in the axial direction of the inside tubular member 28a over the entire length of the inside tubular member 28a. In this embodiment, the inside grooves 32a is placed at substantially the same position as the joint portion 31 with respect to the peripheral direction of the central axis of the inside tubular member 28a.

In FIGS. 1 to 3, the bending portion 20 of this embodiment includes an outside tubular member 28b including the similar configuration as the inside tubular member 28a and fitted over the outer periphery of the inside tubular member 28a. The outside tubular member 28b includes no groove. The dimension of the cylindrical portion 30 and the distance between adjacent tubular portions 30 in the axial direction in the outside tubular member 28b are substantially equal to the dimension of the cylindrical portion 30 and the distance between adjacent cylindrical portions 30 in the axial direction in the inside tubular member 28a.

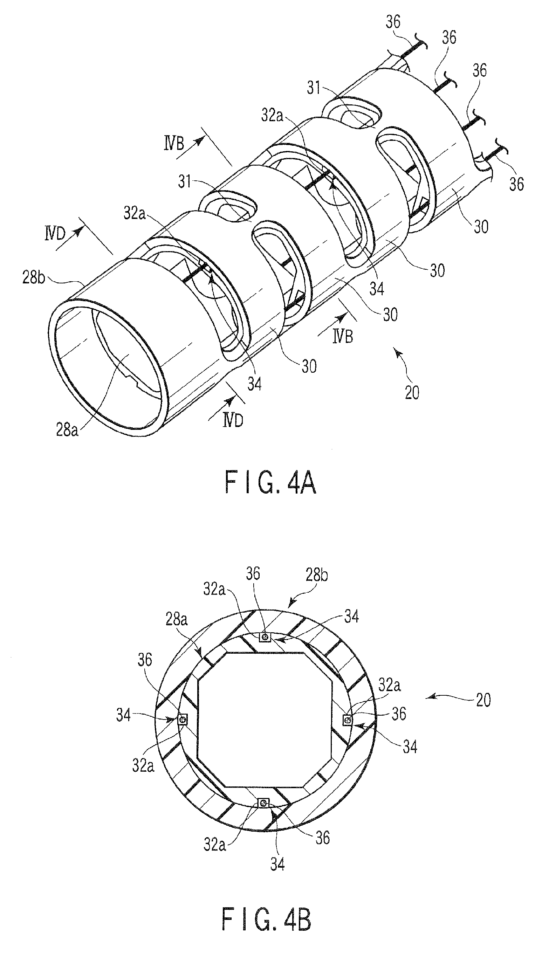

As shown in FIGS. 4A and 4B, the outside tubular member 28b is provided over the outside of and fitted onto the inside tubular member 28a. The inside tubular member 28a and outside tubular member 28b are aligned with respect to the axial direction so that their cylindrical portions 30, 30 are overlapped and aligned with respect to the peripheral direction so that the bending direction of adjacent cylindrical portions 30 of the inside tubular member 28a coincides with the bending direction of adjacent cylindrical portions, overlapping the former, of the outside tubular member 28b. Wire holding portions 34 are formed by the inside groove 32a of the inside tubular member 28a and the inner periphery surface of the outside tubular member 28b at the up, down, left, and right positions in the axial direction of the inside tubular member 28a over the entire length of the inside tubular member 28a. Up, down, left, and right bending operation wires 36 are inserted through these up, down, left and right wire holding portions 34.

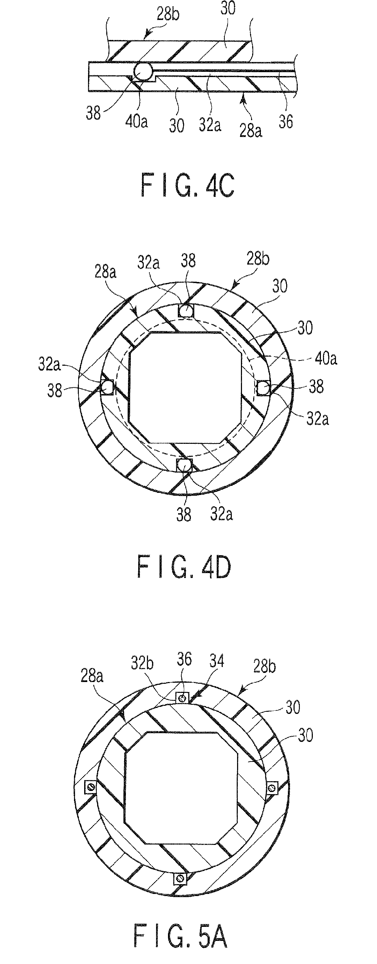

As shown in FIGS. 4C and 4D, the distal end of the operation wire 36 is provided with a spherical fitting portion 38. The fitting portion 38 is fitted into an inside fitting groove 40a formed all around the outer periphery surface of the most forward cylindrical portion 30 of the inside tubular member 28a. The fitting portion 38 is fixed by crimping between the inside fitting groove 40a of the inside tubular member 28a and the inner periphery surface of the most forward cylindrical portion 30.

In FIGS. 1 to 4D, the operation wire 36 is inserted into the control portion 24 through the insertion portion 16, and the proximal end of the operation wire 36 is connected to the bending operation knobs 26a and 26b of the bending operation mechanism. By operating the up/down bending operation knob 26a and left/right bending operation knob 26b, the up, down, left, and right bending operation wires 36 are moved forward or backward, and the bending portion 20 is bent in the up, down, left, and right direction.

Therefore, the endoscope 14 according to this embodiment provides the following effects.

In the bending portion 20 of this embodiment, the cylindrical portions 30 of the inside tubular member 28a and the joint portion 31 connecting the cylindrical portions 30 to be bendable to each other are formed as one body as the inside tubular member 28a. Further, the wire holding portion 34 holding the operation wire 36 to be movable forward and backward between the inside tubular member 28a and outside tubular member 28 is formed by the inside tubular member 28a and outside tubular member 28. Therefore, the bending portion 20 can be very easily manufactured, and the endoscope 14 can be easily manufactured.

In the process of assembling the bending portion 20, the fitting portion 38 of the operation wire 36 is fitted into the inside fitting groove 40a, the operation wire 36 is inserted into each inside groove 32a, from the outside of the inside tubular member 28a and then the outside tubular member 28b is provided over the outside of the inside tubular member 28a. Therefore, the bending portion 20 can be easily assembled.

The wire holding portion 34 is formed by the inside groove 32a extended in the axial direction of the inside tubular member 28a in the outer periphery surface of the inside tubular member 28a and the inner periphery surface of the outside tubular member 28b. Namely, the inside groove 32a is formed in the inside tubular member 28a, but a groove is not formed in the outside tubular member 28b. Therefore, this embodiment is preferable in the bending portion where it is desirable that the inside tubular member 28a is thick and the outside tubular member 28b is thin with regard to the arrangement and demanded strength of the built-in member.

FIGS. 5A to 5C show a second embodiment of the present invention. Components including the same functions as those of the first embodiment are given the same reference numbers, and explanation is omitted.

As shown in FIG. 5A, in this embodiment, a groove is not formed in the inside tubular member 28a. At the up, down, left, and right positions in the inner periphery surface of the outside tubular member 28b, the outside groove 32b is formed in the axial direction of the outside tubular member 28b over the entire length of the outside tubular member 28b. The wire holding portion 34 is formed by the outer periphery surface of the inside tubular member 28a and the outside groove 32 of the outside tubular member 32b.

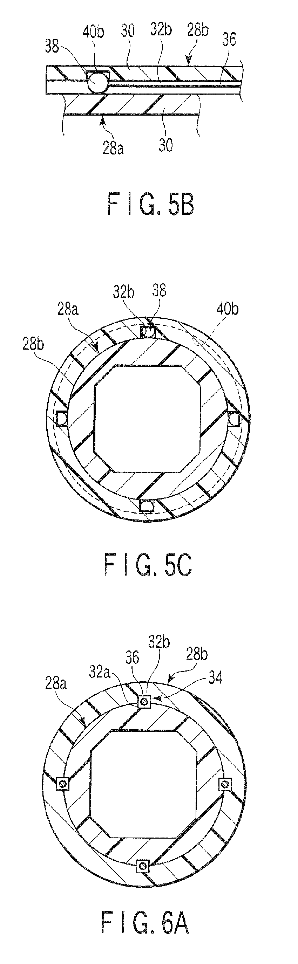

As shown in FIGS. 5B and 5C, an outside fitting groove 40b is formed all around the outer periphery surface of the most forward cylindrical portion 30 of the outside tubular member 28b. The fitting portion 38 of the operation wire 36 is fitted into the outside fitting groove 40b, and fixed by crimping between the outer periphery surface of the most forward cylindrical portion 30 of the outside tubular member 28b and the outside fitting groove 40b of the outside tubular member 28b.

This embodiment is preferable in the bending portion where it is desirable that the inside tubular member 28a is thin and the outside tubular member 28b is thick.

FIGS. 6A to 6C show a third embodiment of the present invention. Components including the same functions as those of the first embodiment are given the same reference numbers, and explanation is omitted.

As shown in FIG. 6A, in this embodiment, the inside groove 32a is formed in the inside tubular member 28a as in the first embodiment, and the outside groove 32b is formed in the outside tubular member 28b as in the second embodiment. These inside groove 32a and outside groove 32b are placed facing to each other. The wire holding portion 34 is formed by the inside groove 32a of the inside tubular member 28a and the outside groove 32b of the outside tubular member 28b.

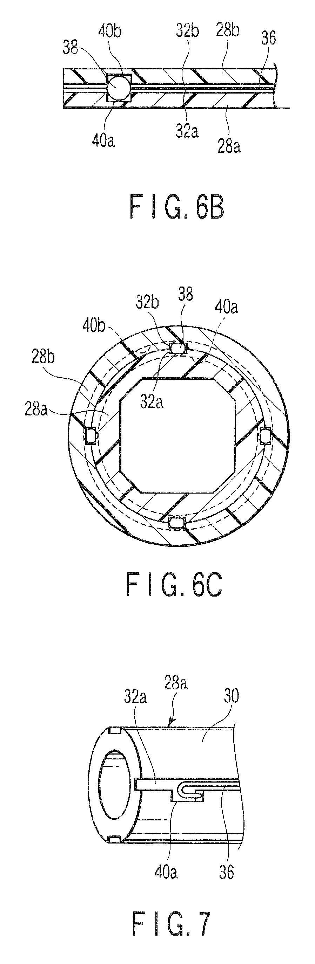

As shown in FIGS. 6B and 6C, the inside fitting groove 40a is formed in the inside tubular member 28a as in the first embodiment, and the outside fitting groove 40b is formed in the outside tubular member 28b as in the second embodiment. These inside groove 40a and outside groove 40b are placed facing to each other. The fitting portion 38 of the operation wire 36 is fixed by crimping between the inside fitting groove 40a of the inside tubular member 28a and the outside fitting groove 40b of the outside tubular member 28b.

This embodiment is preferable in the bending portion where it is desirable that the thickness of the inside tubular member 28a and outside tubular member 28b are same extent.

FIG. 7 shows a fourth embodiment of the present invention. Components including the same functions as those of the first embodiment are given the same reference numbers, and explanation is omitted.

In this embodiment, in the outer periphery surface of the most forward cylindrical portion 30 of the inside tubular member 28a, the inside fitting groove 40a is formed to incorporate with the inside groove 32a so as to enlarge the width of the inside groove 32a. The distal end portion of the operation wire 36 is folded, fitted into the inside fitting groove 40a, and fixed to the inside tubular member 28a. In this embodiment, a part like the fitting portion in the first to third embodiments is unnecessary to fix the distal end portion of the operation wire 36, and the number of parts is reduced.

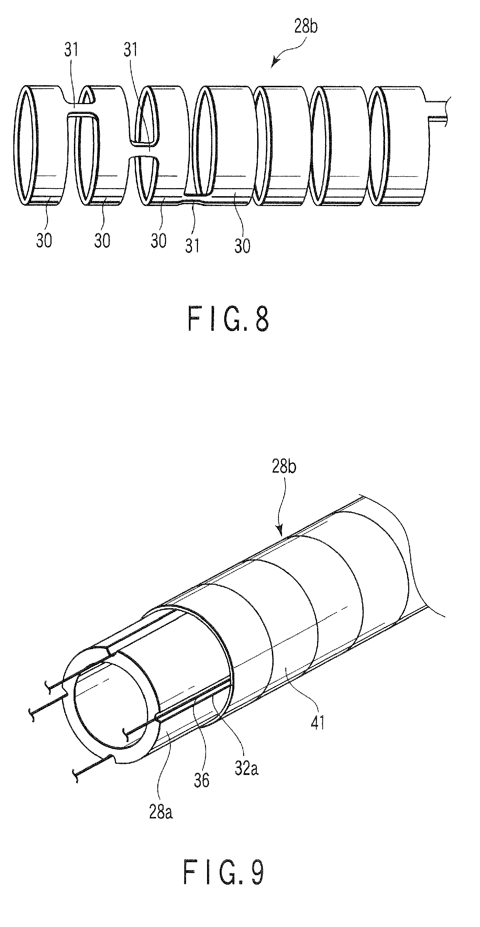

FIG. 8 shows a fifth embodiment of the present invention. Components including the same functions as those of the first embodiment are given the same reference numbers, and explanation is omitted.

In FIG. 8 and FIGS. 1 and 2, in the outside tubular member 28 of this embodiment, adjacent cylindrical portions 30 are connected by one joint portion 31, and one cylindrical portion 30 is bendable in any direction with respect to the other cylindrical portion 30. The joint portion 31 is successively and spirally disposed in the axial direction of the outside tubular member 28b.

In the first embodiment, when the bending direction of adjacent cylindrical portions 30 of the inside tubular member 28a is different from the bending direction of adjacent cylindrical portions 30, overlapping the former, of the outside tubular member 28b, the inside tubular member 28a and outside tubular member 28b interfere each other and the bending is disturbed, and the bending portion 20 becomes difficult to bend. Thus, it is necessary to exactly position the inside tubular member 28a and outside tubular member 28b in the peripheral direction in order to make the bending directions the same.

In contrast to the above, in the outside tubular member 28b of the fifth embodiment, one cylindrical portion 30 is bendable in any direction with respect to the other cylindrical portion 30. Therefore, by positioning the inside tubular member 28a and outside tubular member 28b so as to overlap the mutual cylindrical portions 30, the inside tubular member 28a and outside tubular member 28b rarely interfere each other to disturb the bending and positioning is unnecessary for the inside tubular member 28a and outside tubular member 28b with respect to the peripheral direction. Whereby, the bending portion 20 can be more simply manufactured, and the endoscope 14 can be more easily manufactured.

Although the joint portion of the outside tubular member 28b is spirally arranged along the axial direction of the outside tubular member 28b in this embodiment, the joint portion 31 of the inside tubular member 28a may be spirally arranged.

FIG. 9 shows a sixth embodiment of the present invention. Components including the same functions as those of the first embodiment are given the same reference numbers, and explanation is omitted.

In FIGS. 1 and 9, the insertion portion 16 of this embodiment includes a helical tube 41 over the entire length of the insertion portion 16. The helical tube 41 is formed as one body by spirally winding a beltlike member. The helical tube 41 is used to form the insertion tube portion 22, and forms the outside tubular member 28b in the bending portion 20.

In this embodiment, however the inside tubular member 28a and outside tubular member 28b are arranged to each other with respect to axial direction or peripheral direction, they rarely interfere each other to disturb the bending, and so it is unnecessary to position the inside tubular member 28a and outside tubular member 28b. As one helical tube 41 can be used to form the bending portion 20 and insertion tube portion 22, the simple configuration, easy assembly, and reduced number of parts for the insertion portion 16 of the endoscope 14 is realized.

Although the helical tube 41 forms the outside tubular member 28b in this embodiment, the outside tubular member 28b may be formed by using any tubular member formed as one body, bendable in any direction and capable of forming the wire holding portion 34 between the inside groove 32a of the inside tubular member 28a and itself. Such a tubular member may be formed as one body with an outer member such as a braid tube and a rubber tube. Further, although the helical tube 41 forms the outside tubular member 28b in this embodiment, the helical tube 41 may form the inside tubular member 28a and the outside tubular member 28b of the second embodiment may be used as the outside tubular member 28b.

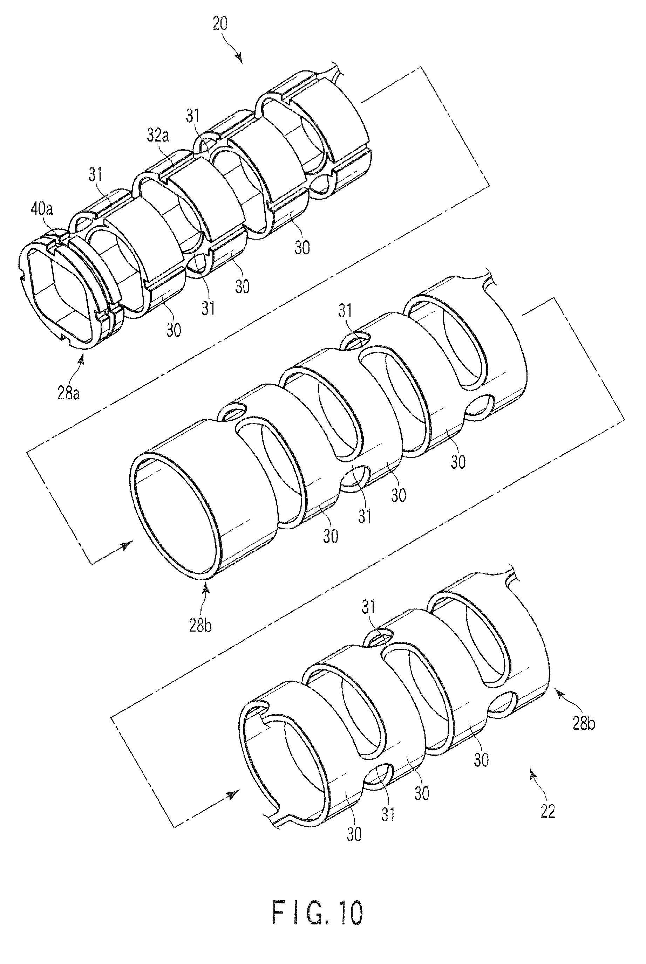

FIG. 10 shows a seventh embodiment of the present invention. Components including the same functions as those of the first embodiment are given the same reference numbers, and explanation is omitted.

In FIGS. 1, 4A and 10, in the insertion portion 16 of this embodiment, the inside tubular member 28a is extended over the bending portion 20, and the outside tubular member 28b is extended over the bending portion 20 and insertion tube portion 22. The operation wire 36 extended from the proximal end of the inside groove 32a of the inside tubular member 28a is inserted through the insertion tube portion 22 into the control portion 24. In the insertion tube portion 22, a coil sheath is provided over the outside of the operation wire 36 for preventing friction between the wire 36 and built-in member. The outside tubular member 28b is covered by an outer tube (not shown) made of flexible resin as one body over the bending portion 20 and insertion tube portion 22.

Therefore, the endoscope 14 according to this embodiment provides the following effects.

In the insertion portion 16 of this embodiment, the inside tubular member 28a is extended over the bending portion 20, and the outside tubular member 28b is extended over the bending portion 20 and insertion tube portion 22. Namely, the portion connecting the bending portion 20 and insertion tube portion 22 is not configured to connect the frame members of the insertion portion 16. Therefore, the configuration is simplified, the number of parts is reduced, the ease of assembly is improved, and the cost of the insertion portion 16 can be downed.

The outer tube of the insertion portion 16 is extended as one body over the bending portion 20 and the insertion tube portion 22. Namely, the bending portion 20 and the insertion tube portion 22 need not be covered with different tubes, and troublesome work such as fixing the outer tube by winding a gut and bonding is unnecessary to ensure airtightness in the portion connecting the bending portion 20 and the insertion tube portion 22. Therefore, the cost of the insertion portion 16 can be reduced still further.

The frame structure of the bending portion 20 of the insertion portion 16 is formed only by fitting the inside tubular member 28a assembled with the operation wire 36 into the outside tubular member 28b, and fixing by crimping the fitting portion 38 of the operation wire 36 between the inside fitting groove 40a of the inside tubular member 28a and the inner periphery surface of the outside tubular member 28b. Therefore, the number of parts is reduced still further, the ease of assembly is improved still further, and the cost of the insertion portion 16 can be sufficiently reduced.

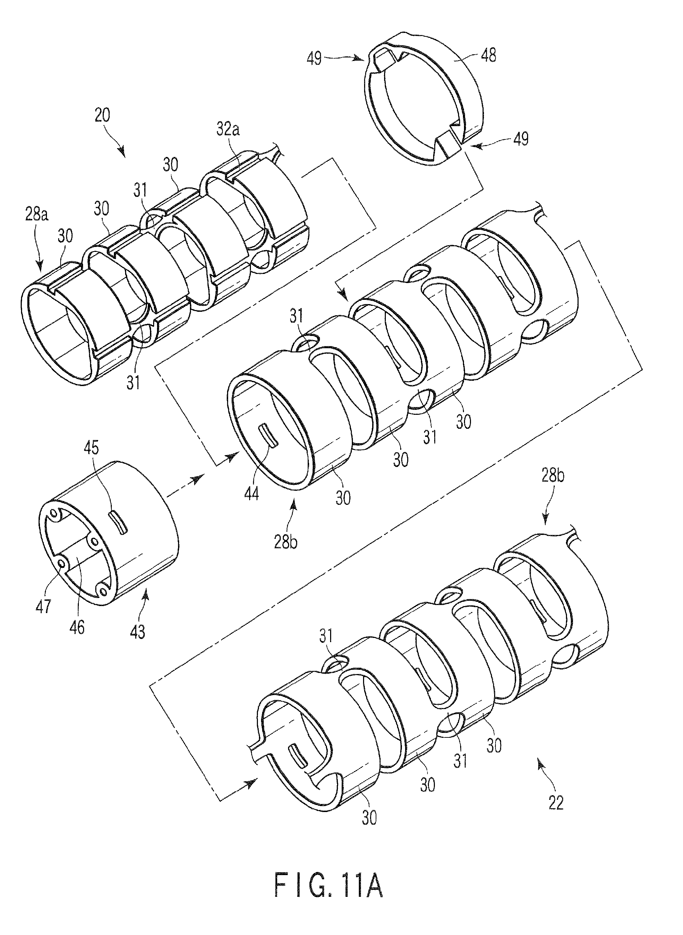

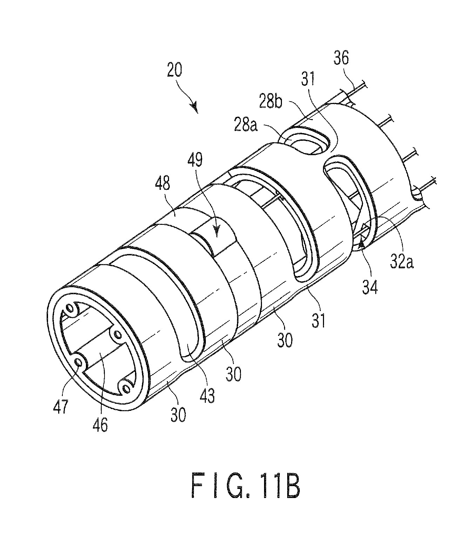

FIGS. 11A and 11B show an eighth embodiment of the present invention. Components including the same functions as those of the seventh embodiment are given the same reference numbers, and explanation is omitted.

In FIGS. 11A and 11B, in the inside tubular member 28a and outside tubular member 28b of this embodiment, the shape of all cylindrical portions 30 including the most forward cylindrical portion 30 is substantially the same. Namely, unlike the seventh embodiment, the inside fitting groove 40a is not formed in the most forward cylindrical portion 30 of the inside tubular member 28a in this eighth embodiment. The inside tubular member 28a and outside tubular member 28b are formed as one body by forming a sufficiently long base member by extrusion molding of elastic resin material or by pressing metal material, and cutting the base member into a desired length.

The most forward cylindrical portion 30 of the inside tubular member 28a is placed at the position of the second cylindrical portion 30 of the outside tubular member 28b from the distal end. A substantially cylindrical boss portion 43 made of resin or metal material such as stainless steel is fitted into the distal end portion of the outside tubular member 28b. The distal end surface of the inside tubular member 28a contact the proximal end surface of the boss portion 43.

A fitting recess 44 is formed in the inner periphery surface of each cylindrical portion 30 of the outside tubular member 28b. A fitting projection 45 of the boss portion 43 is fitted into the fitting recess 44 of the most forward cylindrical portion 30 of the outside tubular member 28b, and the outside tubular member 28b is engaged with the boss portion 43. In this embodiment, since the outside tubular member 28b is formed by cutting a continuously formed base member, the fitting recess 44 is formed in all cylindrical portions 30.

In the inner periphery surface of the boss portion 43, a projection 46 projecting radially and inwardly is provided at the up, down, left, and right positions, and extended in the axial direction. In the projection 46, a wire fixing hole 47 is formed to penetrate in the axial direction. The distal end portion of the inside groove 32a at the up, down, left, and right positions of the inside tubular member 28a is placed facing to the proximal end portion of the wire fixing hole 47 at the up, down, left, and right positions of the boss portion 43. The operation wire 36 extended from the distal end of the inside groove 32a of the inside tubular member 28a is inserted into the wire fixing hole 47, and fixed to the boss portion 43 by bonding, soldering and the like.

The outside tubular member 28b and inside tubular member 28a are engaged with each other by a engaging ring 48 fitted between adjacent cylindrical portions 30 of the outside tubular member 28b. Namely, the cross-section perpendicular to the central axis of the engaging ring 48 is substantially C-shaped. In a clearance portion of the C-shape and a middle portion at the position symmetric to the clearance portion with respect to the central axis, gripping portions 49 for gripping the overlapped joint portions 31 of the outside tubular member 28b and of the inside tubular member 28a is provided. One overlapped joint portions 31 is passed through the clearance portion of the engaging ring 48, and one overlapped joint portions 31 is gripped by the gripping portion 49 in the middle portion of the engaging ring 48 to engage them with each other, while the other overlapped joint portions 31 placed at the position symmetric to one overlapped joint portions 31 with respect to the central axis is held by the gripping portion 49 in the clearance portion of the engaging ring 48 to engage them with each other. Displacement between the outside tubular member 28b and inside tubular member 28a is prevented in this way, and the outside tubular member 28b and inside tubular member 28a can be bent together.

Therefore, the endoscope 14 according to this embodiment provides the following effects.

In the insertion portion 16 of the endoscope of this embodiment, the inside tubular member 28a and outside tubular member 28b are formed by cutting a continuously formed base member into a desired length. Therefore, the inside tubular member 28a and outside tubular member 28b can be speedily manufactured in large quantity at low cost. The cost of the insertion portion 16 of the endoscope can be reduced still further.

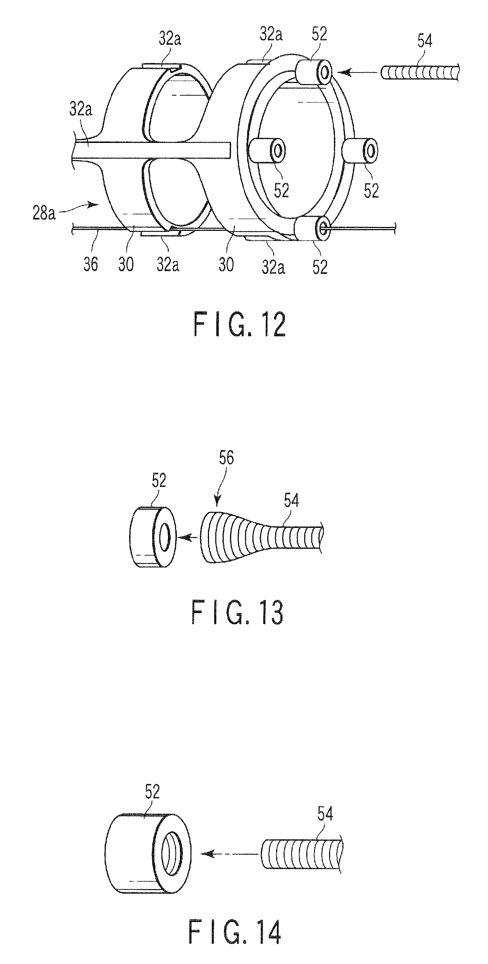

FIG. 12 shows a ninth embodiment of the present invention. Components including the same functions as those of the seventh embodiment are given the same reference numbers, and explanation is omitted.

In the proximal end portion of the inside tubular member 28a of this embodiment, a substantially cylindrical boss 52 is projected in the axial direction of the inside tubular member 28a at up, down, left, and right positions. The distal end of the cavity of the boss 52 is connected to the proximal end of the inside groove 32a. The distal end of coil sheath 54 is inserted into the proximal end of the cavity of the boss 52, and fixed to the boss 52. The operation wire 36 is inserted from the inside groove 32a into the coil sheath 54 through the boss 52.

Therefore, the endoscope 14 according to this embodiment provides the following effects.

In the configuration that the operation wire 36 is directly extended from the proximal end of the inside tubular member 28a, the movement of the operation wire 36 is not stable at the proximal end of the inside tubular member 28a, and the edge of the inside groove 32a of the inside tubular member 28a may be worn by the forward/backward movements of the operation wire 36. In the insertion portion 16 of the endoscope of this embodiment, the operation wire 36 is inserted from the inside groove 32a into the coil sheath 54 through the boss 52, and the above unstable movement and wear are prevented.

FIG. 13 shows a first modification of the ninth embodiment of the present invention. Components including the same functions as those of the ninth embodiment are given the same reference numbers, and explanation is omitted. In this modification, a tapered portion 56 with the diameter gradually increased to the distal end side is formed at the distal end of the coil sheath 54. This tapered portion 56 is provided over and fixed to the outer periphery surface of the boss 52.

FIG. 14 shows a second modification of the ninth embodiment of the present invention. Components including the same functions as those of the ninth embodiment are given the same reference numbers, and explanation is omitted. In this modification, a female screw is formed in the inner periphery surface of the boss 52. A winding at the distal end of the coil sheath 54 functions as a male screw. The distal end of the coil sheath 54 is screwed to the boss 52.

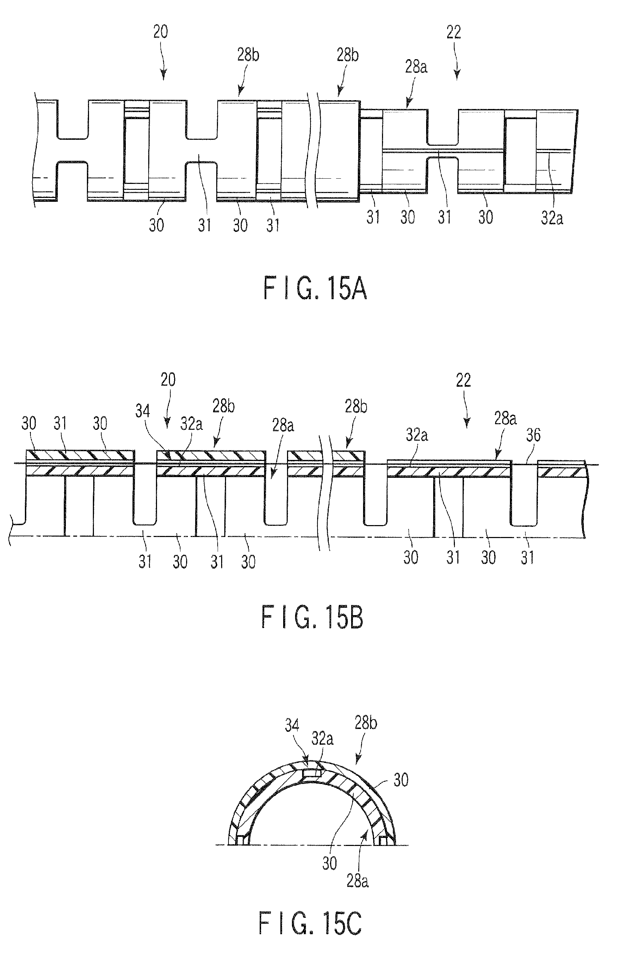

FIGS. 15A to 15C show a tenth embodiment of the present invention. Components including the same functions as those of the seventh embodiment are given the same reference numbers, and explanation is omitted.

In this embodiment, the outside tubular member 28b is extended over the bending portion 20, and the inside tubular member 28a is extended over the bending portion 20 and the insertion tube portion 22. In the outer periphery surface of the inside tubular member 28a, the inside groove 32a is extended over the entire length of the inside tubular member 28a. In the bending portion 20 and the insertion tube portion 22, the operation wire 36 is inserted and held to be movable forward and backward in the inside groove 32a. An outer tube (not shown) made of flexible resin is covered as one body over the bending portion 20 and the insertion tube portion 22.

Therefore, the endoscope 14 according to this embodiment provides the following effects.

Usually, the outside diameter of the insertion portion 16 is smaller, smoothness of insertion of the endoscope insertion portion 16 into a patient's body cavity is more improved. The inside diameter in the bending portion 20 is larger, the built-in member can be moved more smoothly when the bending portion 20 is bent, friction between the internal tubes is more prevented, and durability of the internal tubes is more improved.

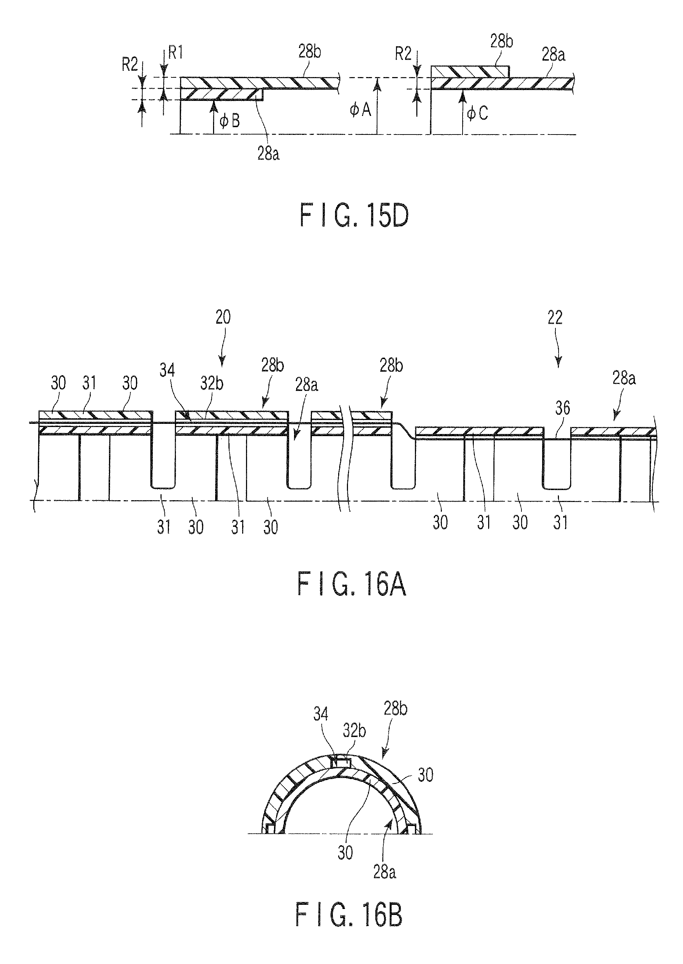

The case is considered that smoothness of insertion is set constant, that is, the outside diameter of the frame member in the insertion tube portion 22 is set to constant φA. As the length of the bending portion 20 in the axial direction is sufficiently shorter than the length of the insertion tube portion 22 in the axial direction, smoothness of insertion of the whole insertion portion 16 into the body cavity is almost not affected even if the outside diameter of the bending tube 20 is a little larger than the outside diameter of the insertion tube portion 22. As shown in FIG. 15D, as in the seventh to ninth embodiments, when the outside tubular member 28b is extended over the bending portion 20 and the insertion tube portion 22, the inside diameter φB of the frame member in the bending portion 20 is obtained by subtracting the thickness R1 of the outside tubular member 28b and the thickness R2 of the inside tubular member 28a from the outside diameter φA of the outside tubular member 28b (φB = φA - 2R1 - 2R2). On the other hand, when the inside tubular member 28 is extended over the bending portion 20 and the insertion tube portion 22 as in this embodiment, the inside diameter φC of the frame member in the bending portion 20 is obtained by subtracting only the thickness R2 of the inside tubular member 28a from the outside diameter φA of the inside tubular member 28a (φC = φA - 2R2). Namely, when the inside tubular member 28a is extended over the bending portion 20 and the insertion tube portion 22, the inside diameter of the frame member in the bending portion 20 can be larger, compared with the case that the outside tubular member 28b is extended over the bending portion 20 and the insertion tube portion 22.

As described above, this embodiment can simultaneously realize excellent smoothness of insertion of the insertion portion 16 and excellent durability of the built-in member.

FIGS. 16A and 16B show a modification of the tenth embodiment of the present invention. Components including the same functions as those of the tenth embodiment are given the same reference numbers, and explanation is omitted.

In this embodiment, the inside groove 32a is not formed in the inside tubular member 28a. In the inner periphery surface of the outside tubular member 28b, the outside groove 32b is formed in the axial direction of the outside tubular member 28b at up, down, left, and right positions. The wire holding portion 34 is formed by the outside groove 32b and the outer periphery surface of the inside tubular member 28a. In the proximal end portion of the outside tubular member 28b, the operation wire 36 is extended from the proximal end of the outside groove 32b, taken into the inside tubular member 28a through between adjacent cylindrical portions 30 of the inside tubular member 28a, and reached the control portion 24 through the inside tubular member 28a.

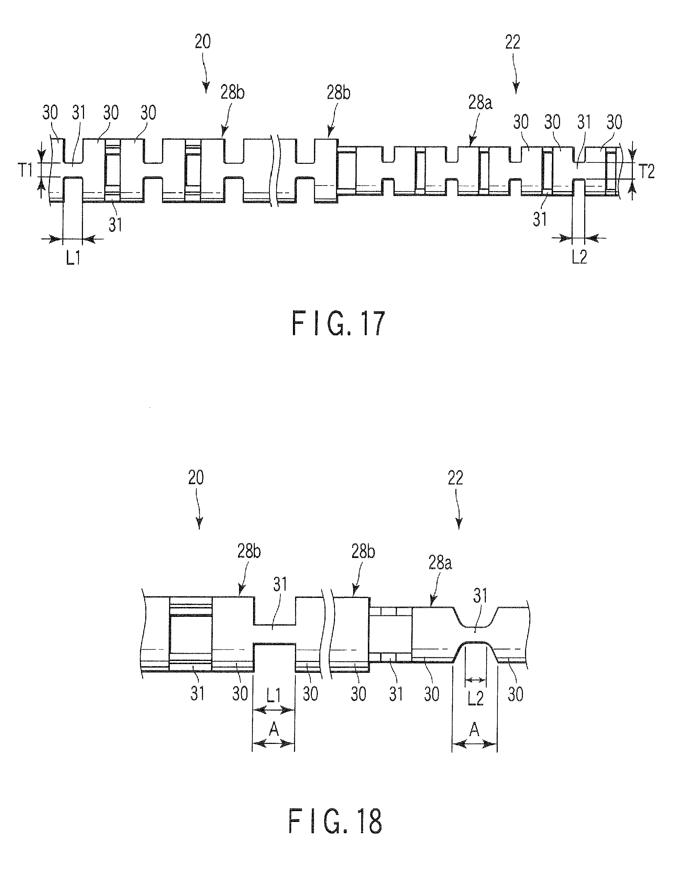

FIG. 17 shows an eleventh embodiment of the present invention. Components including the same functions as those of the tenth embodiment are given the same reference numbers, and explanation is omitted.

In this embodiment, in the joint portion 31 connecting adjacent cylindrical portions 30, the length in the axial direction (interval between adjacent cylindrical portions 30) and the width in the peripheral direction are changed between the bending portion 20 and the insertion tube portion 22. Namely, the length L1 of the joint portion 31 in the bending portion 20 is longer than the length L2 of the joint portion 31 in the insertion tube portion 22. The width T1 of the joint portion 31 in the bending portion 20 is narrower than the width T2 of the joint portion 31 in the insertion tube portion 22. Therefore, the flexibility of the joint portion 31 in the bending portion is higher than that of the joint portion 31 in the insertion tube portion 22 and so the flexibility of the frame member in the bending portion 20 is higher than that in the insertion tube portion 22.

Therefore, the endoscope 14 according to this embodiment provides the following effects.

When the flexibility of the frame member in the bending portion 20 is not higher than that in the insertion tube portion 22, the bending performance may be downed, for example, when the operation wire is moved forward or backward, insertion tube portion 22 is unexpectedly bent together with the bending portion 20 or the bending portion 20 is difficult to bend while the insertion tube portion 22 is not bent. In this embodiment, the flexibility in the frame member in the bending portion 20 is higher than that in the insertion tube portion 22, and degradation in the bending performance is prevented.

FIG. 18 shows a first modification of the eleventh embodiment of the present invention. Components including the same functions as those of the eleventh embodiment are given the same reference numbers, and explanation is omitted.

In this embodiment, the interval A between adjacent cylindrical portions 30 in the bending portion 20 and the insertion tube portion 22 is constant. In the insertion tube portion 22, both proximal ends of the joint portion 31 is tapered with the width gradually increased to the cylindrical portion 30 in order to reduce the effective length of the joint portion 31 contributive to the bending. Namely, as shown in FIG. 18, when the effective length of the joint portion 31 of the bending portion 20 is set to L1 and the effective length of the joint portion 31 of the insertion tube portion 22 is set to L2, L1 < L2.

A twelfth embodiment of the present invention will now be explained. Components including the same functions as those of the eleventh embodiment are given the same reference numbers, and explanation is omitted.

In this embodiment, in the bending portion 20, the length of each joint portion 31 is gradually reduced and the width of each joint portion is increased, from the distal end side to the proximal end side. Namely, in the bending portion 20, the flexibility of the frame member is reduced from the distal end side to the proximal end side.

Therefore, the endoscope 14 according to this embodiment provides the following effects.

In the bending portion 20, when the flexibility is constant over the entire length, the curvature at the time of bending is substantially constant over the entire length. In this case, the insertion portion 16 is extremely deformed in the portion connecting the bending portion and the insertion tube portion 22, and the insertion portion 16 may be damaged by concentration of stress. In this embodiment, the flexibility of the frame member is reduced from the distal and side to the proximal end side in the bending portion 20, and the curvature at the time of bending the bending portion 20 is also reduced from the distal end side to the proximal end side. Therefore, the deformation of the insertion portion 16 is gradual in the portion connecting the bending portion 20 and insertion tube portion 22, the concentration of stress is avoided, and the damage of the insertion portion 16 can be prevented.

A thirteenth embodiment of the present invention will now be explained. Components including the same functions as those of the tenth embodiment are given the same reference numbers, and explanation is omitted.

In this embodiment, the thickness of the outer tube to cover the frame member is changed between the bending portion 20 and insertion tube portion 22. Namely, the outer tube thickness in the bending portion 20 is thinner than that in the insertion tube portion 22. Therefore, the flexibility of the bending portion 20 is higher than that of the insertion tube portion 22, and degradation in the bending performance is prevented as in the eleventh embodiment.

## Claims

1. An endoscope insertion portion **characterized by** comprising:
an inside tubular member shaped substantially a cylinder, and extended over at least a bending portion;
an outside tubular member shaped substantially a cylinder, provided over an outside of the inside tubular member, and extended over at least a bending portion; and
an operation wire for bending operation of the inside tubular member and outside tubular member;
wherein at least one tubular member of the inside tubular member and outside tubular member is formed as one body, and includes at least two cylindrical portions and a joint portion including elasticity and connecting the adjacent cylindrical portions to be bendable to each other, and
a wire holding portion is formed by the inside tubular member and outside tubular member and holds the operation wire to be movable forward and backward between the inside tubular member and outside tubular member.

2. The endoscope insertion portion according to claim 1, **characterized in that** the other tubular member of the inside tubular member and outside tubular member is formed as one body, and includes at least two cylindrical portions and a joint portion including elasticity and connecting the adjacent cylindrical portions to be bendable to each other.

3. The endoscope insertion portion according to claim 1, **characterized in that** the wire holding portion is formed by a groove formed on the outer periphery surface of the inside tubular member and extended in a central axial direction of the inside tubular member and the inner periphery surface of the outside tubular member.

4. The endoscope insertion portion according to claim 1, **characterized in that** the wire holding portion is formed by the outer periphery surface of the inside tubular member and a groove formed on the inner periphery surface of the outside tubular member and extended in a central axial direction of the outside tubular member.

5. The endoscope insertion portion according to claim 1, **characterized in that** the wire holding portion is formed by an inside groove formed on the outer periphery surface of the inside tubular member and extended in a central axial direction of the inside tubular member and an outside groove formed on the inner periphery surface of the outside tubular member, extended in a central axial direction of the outside tubular member, and facing to the inside groove.

6. The endoscope insertion portion according to claim 2, **characterized in that** the joint portions of the other tubular member are successively and helically arranged in a central axial direction of the other tubular member.

7. The endoscope insertion portion according to claim 1, **characterized in that** the other tubular member of the inside tubular member and outside tubular member is formed by a helical tube formed as one body.

8. The endoscope insertion portion according to claim 7, **characterized in that** the helical tube is extended over the bending portion and the insertion tube portion.

9. The endoscope insertion portion according to claim 2, **characterized in that** the one tubular member of the inside tubular member and outside tubular member is extended over the bending portion and the other tubular member of the inside tubular member and outside tubular member is extended over the bending portion and the insertion tube portion.

10. The endoscope insertion portion according to claim 9, **characterized in that** the inside tubular member is formed by cutting a base member including cylindrical portions provided coaxially to each other and joint portions connecting the adjacent cylindrical portions to be bendable to each other and formed continuously, into a predetermined length.

11. The endoscope insertion portion according to claim 9, **characterized in that** the outside tubular member is formed by cutting a base member including cylindrical portions provided coaxially to each other and joint portions connecting the adjacent cylindrical portions to be bendable to each other and formed continuously, into a predetermined length.

12. The endoscope insertion portion according to claim 9, **characterized in that**
the inside tubular member is extended over the bending portion,
the outside tubular member is extended over the bending portion and the insertion tube portion,
the operation wire is inserted through the bending portion and the insertion tube portion,
the endoscope insertion portion further comprises a sheath provided over an outside of the operation wire in the insertion tube portion,
the inside tubular member includes at least one boss provided in a proximal end portion of the inside tubular member, connected to the wire holding portion, and connected to a distal end of the sheath, and
the operation wire is inserted from the wire holding portion into the sheath through the boss.

13. The endoscope insertion portion according to claim 9, **characterized in that**
the inside tubular member is extended over the bending portion and the insertion tube portion, and
the outside tubular member is extended over the bending portion.

14. The endoscope insertion portion according to any one of claims 9 to 13, **characterized by** further comprising an outer tube formed as one body and extended over the bending portion and the insertion tube portion.

15. The endoscope insertion portion according to any one of claims 9 to 13, **characterized in that** a length of the joint portion in the longitudinal axial direction of the tubular member in the bending portion is longer than a length of the joint portion in the longitudinal axial direction of the tubular member in the insertion tube portion.

16. The endoscope insertion portion according to any one of claims 9 to 13, **characterized in that** a width of the joint portion in a peripheral direction of the tubular member in the bending portion is narrower than a width of the joint portion in the peripheral direction of the tubular member in the insertion tube portion.

17. The endoscope insertion portion according to claim 14, **characterized in that** a thickness of the outer tube in the bending portion is thinner than a thickness of the outer tube in the insertion tube portion.

18. An endoscope **characterized by** comprising the endoscope insertion portion according to any one of claims 1 to 17.
